# EUROPEAN PATENT SPECIFICATION

(11) **EP 4 048 276 B1**
(45) Date of publication and mention of the grant of the patent: **03.01.2024**
(21) Application number: 19949748.8
(22) Date of filing: 26.10.2019
(51) Int. Cl.: A61K 31/519, A61K 9/20

(54) **SOLID PHARMACEUTICAL FORMULATIONS COMPRISING TICAGRELOR**
FESTE PHARMAZEUTISCHE FORMULIERUNGEN MIT TICAGRELOR
FORMULATIONS PHARMACEUTIQUES SOLIDES COMPRENANT DU TICAGRELOR

(43) Date of publication of application: 31.08.2022
(73) Proprietor: Santa Farma Ilaç Sanayi A.S., 34382 Istanbul (TR)
(72) Inventor: YILDIRIM, Ersin, 41455 Dilovas /Kocaeli (TR); KANIK, Bayram, 41455 Dilovas /Kocaeli (TR); ÖZTÜRK, Fatma, 41455 Dilovas /Kocaeli (TR); AKTAS , Tansel, 41455 Dilovas /Kocaeli (TR)
(74) Representative: Bulut, Pinar
(86) International application number: PCT/TR2019/050903
(87) International publication number: WO 2021/080529

(56) References cited:
- EP-A1- 3 332 769
- EP-A1- 3 332 769
- WO-A1-2019/170244
- US-A1- 2017 296 666
- LI J Z ET AL: "The role of intra- and extragranular microcrystalline cellulose in tablet dissolution", PHARMACEUTICAL DEVELOPMENT AND TECHNOLOGY, NEW YORK, NY, US, vol. 1, no. 4, 1 December 1996 (1996-12-01), pages 343-355, XP009124869, ISSN: 1083-7450, DOI: 10.3109/10837459609031429
- LI, J. Z. et al.: "The role of intra-and extragranular microcrystalline cellulose in tablet dissolution", Pharmaceutical development and technology, vol. 1, no. 4, 1996, pages 343-355, XP009124869, DOI: 10.3109/10837459609031429

## Description

### FIELD OF INVENTION

The present invention relates to a pharmaceutical composition, comprising ticagrelor, wherein the pharmaceutical composition is in a solid dosage form with improved pharmaceutical characteristics.

### STATE OF ART

Ticagrelor is a platelet aggregation inhibitor that acts by inhibition of adenosine 5-diphosphate. Ticagrelor is a reversible, selective P2Y12-receptor antagonist. Its chemical name is (1S,2S,3R,5S)-3-(7-{[(1R,2S)-2-(3,4-Diflourophenyl)cyclopropyl] amino}-5-(propylsulfanyl)-3H-[1,2,3]triazol[4,5-d]pyrimidine-3-yl)-5-(2-hydroxyethoxy)cyclopentane-1,2-diol and its molecular structure is given below. Its CAS Number is 274693-27-5.

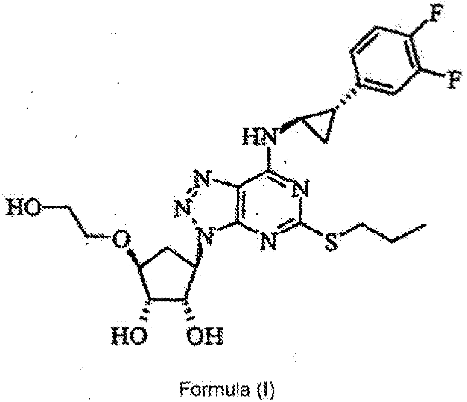

Ticagrelor is marketed by AstraZeneca under trade names BRILINTA and BRILIQUE with 60 mg and 90 mg in worldwide. Ticagrelor is indicated for the treatment or prevention of thrombotic events e.g. stroke, heart attack, acute coronary syndrome or myocardial infarction with ST elevation, other coronary artery diseases and arterial thrombosis plus other disorders related to platelet aggregation (WO 00/34283).

WO99/05143 discloses a series of triazolo [4,5-d]pyrimidine compounds, including Ticagrelor. WO01/92262 and WO2013/079589 disclose various crystalline and amorphous forms of Ticagrelor. In the pharmaceutical dosage forms of the present invention any of these forms can be used.

Pharmaceutical compositions comprising ticagrelor are disclosed in WO 2008/024044 and WO 2008/024045. Oral pharmaceutical compositions which can comprise up to 50% by weight of the active ingredient are explained and they release all the active ingredient substantially. A pharmaceutical composition comprises active ingredient, a filler consisting essentially of a mixture of mannitol and dibasic calcium phosphate dihydrate, a binder consisting essentially of hydroxypropyl cellulose, a disintegrant consisting essentially of sodium starch glycolate, and one or more lubricants. Wet granulation process is preferred as the manufacturing method.

WO2015001489 relates to oral pharmaceutical compositions comprising amorphous ticagrelor and processes for their preparation.

TR2017/15203 discloses a method of manufacture for a formulation comprising ticagrelor, characterized in that the step of granulating the solid mixture wherein the mixture comprises the active agent ticagrelor and at least one water-soluble binder and at least one water-soluble filler that is granulated with a suitable granulation solution.

TR2016/01835 discloses a process for producing a formulation comprising ticagrelor or a pharmaceutically acceptable salt thereof, characterized in that the ticagrelor or pharmaceutically acceptable salt is dissolved and / or dispersed in a solvent or solvent mixture during manufacture.

EP3332769 relates to a solid oral pharmaceutical composition, comprising ticagrelor or a pharmaceutically acceptable salt thereof with at least one hydrophilic polymer wherein the composition is substantially free of calcium salts and their derivatives.

US2017296666A describes a solid pharmaceutical composition of ticagrelor comprising microcrystalline cellulose and colloidal silicon dioxide in the extragranular phase.

WO2014059955 relates to a solid oral pharmaceutical formulation containing ticagrelor as the active pharmaceutical constituent wherein the pharmaceutical composition contains at least one the non-hygroscopic binder, at least one the non-hygroscopic filler and at least one lubricant and substantially no disintegrant.

WO2018229785 relates to oral pharmaceutical compositions comprising ticagrelor and pharmaceutically acceptable polymer in a ratio of 1:0.1 to 1:1 in the form of solid dispersion.

WO2015110952 relates to a composition comprising ticagrelor or salt thereof in an amount less than 20 % of the weight of total composition, wherein the composition is devoid of water- insoluble fillers

CN107397717 discloses a solid preparation of ticagrelor or a pharmaceutically acceptable salt thereof which comprises filler, disintegrant, lubricant, additive and high polymer. The solid preparation is prepared by mixing a filler and ticagrelor or its pharmacy or it is acceptable to salt, adding a lubricant and a disintegrant respectively and mixing the additive and the polymer to form a suitable solid preparation.

CN109700773 relates to a pharmaceutical composition comprising ticagrelor wherein the percentage the ticagrelor of weight at the formulation composition is 2%.

CN201310432438 discloses a solid dispersion comprising ticagrelor in which ticagrelor is dispersed in a carrier material to solve the problem of solubility.

CN106265557 discloses a dispersible tablet formulation comprising ticagrelor in which ticagrelor is in an amount of the total tablet weight 20 to 50% by weight.

Ticagrelor is a cyclo-pentyltriazolo-pyrimidine that reversibly inhibits the P2Y12receptor which is a protein found mainly but not exclusively on the surface of blood platelets and which is an important regulator in blood clotting.

Ticagrelor (BRILINTA^{®}) is indicated to reduce the rate of cardiovascular death, myocardial infarction, and stroke in patients with acute coronary syndrome (ACS) or a history of myocardial infarction (MI). For at least the first 12 months following ACS, it is superior to clopidogrel. It also reduces the rate of stent thrombosis in patients who have been stented for treatment of ACS. In the management of ACS, ticagrelor treatment is performed with a 180 mg loading dose. During the first year after an ACS event, 180 mg dose is administered daily. After one year 120 mg dose is administered.

In the state of the art, the patent application no. EP2056832 discloses an oral pharmaceutical composition comprising ticagrelor and other excipients. The composition is presented as enhancing the bioavailability through increasing the release of the active agent due to the formulation comprising at least two fillers and one single binder. Moreover, one of these fillers is dibasic calcium phosphate dehydrate which is commonly known by its abrasive properties. Considering these undesirable properties, the use of dibasic calcium phosphate dehydrate as a tablet excipient shall increase the required amount of lubricants (Handbook of Pharmaceutical Excipients, sixth edition, page 97). However, the ratio of the lubricant given in the application, which is preferably magnesium stearate, is in the range of 0.5-1% by weight. This low ratio is likely to cause stability problems in the presence of dibasic calcium phosphate dehydrate. On the other hand, the increase of the magnesium stearate amount shall reduce the dissolution rate and the promised enhanced bioavailability accordingly (Handbook of Pharmaceutical Excipients, sixth edition, page 405).

Another patent application no. WO2011076749 discloses solid dosage forms comprising ticagrelor, characterized in that at least 90% by volume of ticagrelor particles have a particle size in the range of 1 µm to 150 µm. It further discloses that ticagrelor, being poorly soluble, presents a significant problem in the design of pharmaceutical compositions. Further, in order to exhibit good bioavailability, it is desirable to formulate dosage forms showing fast dissolution of the drug. However, it is stated that these effects are observable only if the ratio of ticagrelor is more that 50% by weight in the composition with the particle size of the drug and by the use of hydrophilic polymers/emulsifiers in the dosage form. Ticagrelor is classified as a BCS class IV compound, exhibiting low solubility and low permeability. This property leads to an undesirable dissolution profile and hence poor bioavailability. It also leads to high intra-subject and inter-subject variability following oral administration.

Ticagrelor has a low aqueous solubility (10 µg/mL in water) and this situation brings bioavailability and stability problems regarding decreases in the solubility of the tablet formulations. In the state of the art, several methods are followed, different forms of ticagrelor and different excipients are used to compose innovative formulations and to overcome some of these problems. However, the challenge is to formulate a composition, some qualities of which won't reduce, while the others are improved. Thus, there is a need in the art for a pharmaceutical composition comprising ticagrelor providing enhanced dissolution profile, stability and bioavailability at the same time.

### SUMMARY OF INVENTION

The object of this invention is to develop an immediate-release solid pharmaceutical formulation comprising ticagrelor or a pharmaceutically acceptable salt thereof, and excipients as described in present claim 1, which has improved dissolution release and blend properties prior to tablet compression.

The present invention relates to an immediate release solid oral composition comprising only ticagrelor as active substance and excipients as described in present claim 1 manufactured by using optimised wet granulation process.

As described herein but not claimed is an oral dosage form composition in concurrent use of acetylsalicylic acid for the treatment of the prevention of atherothrombotic events in adult patients with
- acute coronary syndromes (ACS) or
- a history of myocardial infarction (MI) and a high risk of developing an atherothrombotic event.

Another object of the present invention is to provide a preparation method of a pharmaceutical composition of ticagrelor wherein the pharmaceutical compositions herein disclosed can be manufactured into solid dosage forms, such as tablet, film-coated tablet, capsule, sachet, having good blend characteristics and the desired dissolution profiles.

In a preferred embodiment present invention, the active agent is crystalline ticagrelor. Preferably, the active agent is ticagrelor polymorph II presenting X-ray powder diffraction pattern containing specific peaks at 5.5° (±0.1°), 6.8° (±0.1°), 10.6° (±0.1°), 13.5° (±0.1°), 14.9° (±0.1°), 18.3° (±0.1°), 19.2° (±0.1°), 22.7° (±0.1°), 24.3° (±0.1°) and 27.1° (±0.1°) 2θ.

In a preferred embodiment present invention, the active agent is ticagrelor polymorph II which is also used in innovator drug product and declared that it is the most stable crystalline form of ticagrelor hat has desired solubility characteristics.

Another object of the present invention is to provide a pharmaceutical compositions containing ticagrelor by using wet granulation process wherein provided for the manufacture of solid dosage form containing the active ingredient, and excipients as described in present claim 1.

In another object of the present invention is provided a pharmaceutical compositions containing ticagrelor wherein the total weight of the ticagrelor is 20-30% w/w by total solid dosage form weight.

In another object of the present invention is provided a pharmaceutical composition containing ticagrelor with optimized amounts of excipients manufactured by using wet granulation method.

### DETAILED DESCRIPTION OF THE INVENTION

The present invention provides a pharmaceutical composition comprising ticagrelor and pharmaceutically acceptable excipients manufactured by using wet granulation method wherein;
the composition comprises
- Microcrystalline cellulose used in extragranular phase is between 20-30%w/w by total weight of the formulation,
- Silicon dioxide, wherein the quantitative composition in w/w % is as stated below:
   Ticagrelor 20-30
   Mannitol 30 - 40
   Microcrystalline cellulose 30-40 Hydroxypropyl cellulose 0.1 -5
   Sodium starch glycolate 0.1 -5
   Silicon dioxide 0.1 -2
   Magnesium stearate 0.1 -2
   Deionised water Quantity sufficient Coating agent 1-5
   wherein together in the extragranular phase in weight ratio of Microcrystalline cellulose : Silicon dioxide is between 35:1 to 45:1.

The present invention also provides a wet granulation process for preparing a pharmaceutical composition comprises crystalline Ticagrelor as polymorph II, wherein the process comprises the following steps:
a. Ticagrelor, mannitol, specified amount of microcrystalline cellulose, specified amount of hydroxypropyl cellulose and sodium starch glycolate are stirred in high-shear wet granulator for 5 minutes, b. Granulation solution was prepared by dissolving specified amount of hydroxypropyl cellulose in deionized water,
c. Sufficient quantity of granulation solution was added on the powder blend in step a and granulation process was performed with a 10 minute period stirring,
d. Granule obtained in step c is dried at a specified temperature,
e. Dried granule was sifted and remaining amounts of microcrystalline cellulose and hydroxypropyl cellulose and also silicon dioxide were added and stirred,
f. Obtained granule in step e was lubricated with magnesium stearate for 4 minutes,
g. Final blend in step f is subjected to tablet compression process, 1 h. Tablets obtained in step g were coated with a proper film coating agent.

The present invention provides a solid oral dosage form comprising ticagrelor and optimized amounts of pharmaceutically acceptable excipients as described above manufactured by using the most proper manufacturing method.

The present invention relates to an immediate release solid oral composition comprising ticagrelor and one or more acceptable excipients manufactured by using optimised wet granulation process.

Another object of the present invention is to provide a preparation method of a pharmaceutical composition of ticagrelor wherein the pharmaceutical compositions herein disclosed can be manufactured into solid dosage forms, such as tablet, film coated tablet, sachet, capsule having improved granule characteristics and the desired dissolution profiles.

For illustrative purposes, but not being part of the invention, there is a disclosure of a pharmaceutical composition in general containing ticagrelor by using wet granulation process wherein provided for the manufacture of tablets containg the active ingredient, fillers, diluents, disintegrants, solubilizers, and lubricants etc.

For illustrative purposes, but not being part of the invention, there is a disclosure of a pharmaceutical composition in general containing ticagrelor wherein the total weight of the ticagrelor is less than 30% w/w by total tablet weight.

Basicly three types of manufactruing methods are available in the prior art. Advantageous and disadvantageous of the manufacturing methods are evaluated based on the information and experimental data procured from prior art documents.

Quality by Design (QbD) principles were applied to the development of the ticagrelor comprising pharmaceutical composition. The critical quality attributes that have the potential to affect product performance have been identified using the principles as outlined in ICH Q8 and Q9.

Solubility of ticagrelor is very low (not ionised in the pysiological pH range) and exhibits a moderate intrinsic permeability, there is potentially a higher risk that changes in formulation and processing parameters can affect clinical performance, and this was taken into account during development. Moreover, this low aqueous solubility of ticagrelor leads to an increase of relevance of particle size. Solubility studies in human intestinal fluids, bulk-tapped density, compressibility index, Hausner ratio, flowability studies of drug substance were performed prior to beginning of the development process of pharmaceutical drug product.

| **Ticagrelor** | **Water (mg/mL)** | **pH: 1.2 (mg/mL)** | **pH: 4.5 (mg/mL)** | **pH**: **6.8 (mg/mL)** |
|---|---|---|---|---|
| Average solubility | 0.006 | 0.014 | 0.006 | 0.005 |

As shown in table above, ticagrelor solubility is very low in pysiological pH range.

In addition, bulk density and tapped density values of ticagrelor was investigated. By using the results obtained, compressibility index, Hausner ratio and flowability of drug substance was designated.

| **Ticagrelor** | **Bulk density** | **Tapped density** | **Compressibility index** | **Hausner ratio** | **Flowability** |
|---|---|---|---|---|---|
| Average solubility | 0.157 | 0.257 | 39% | 1.639 | Very poor |

Drug substances with poor aqueous solubility and poor dissolution in the GI fluids is a limiting factor to the in vivo bioavailability after oral administration. Therefore, in vitro dissolution has been recognized as an important element in drug development and thus increasing the dissolution rate of poorly soluble drugs and enhancing their bioavailability is an important challenge to formulation designation studies.

Ticagrelor containing pharmaceutical drug products are designed based on all investment studies explained above. Micronizing particle size values and designation of polymorphism are the main approaches to improve physicochemical and biopharmaceutical properties of the poorly soluble drugs, thereby improving their solubility. These approaches are used for ticagrelor also.

Moreover, particle size distribution analysis for ticagrelor was performed. Results are shown in the table below.

| **D10** | **D50** | **D90** |
|---|---|---|
| 3.0 µm | 6.4 µm | 12.5 µm |

Polymorphism is a direct impact on physical and chemical properties of drug substance. Based on this information XRD anaysis was performed and confirmed that the drug substance used was polymorph II as it is in the innovator drug product.

Non-limiting examples of fillers, as described for understanding the invention, but not being part of the invention, comprise dibasic calcium phosphate dihydrate, polysaccharides, lactose, mannitol, sugars, sorbitol, sucrose, inorganic salts, primarily calcium salts and the like and mixtures thereof. The filler of present invention is microcrystalline cellulose.

Examples of disintegrants, as described for understanding the invention, but not being part of the invention, are croscarmellose sodium, crospovidone, low-substituted hydroxypropyl cellulose, starch, carmellose and mixtures thereof.

The disintegrant of present invention is sodium starch glycolate.

Non-limiting examples of diluents as described for understanding the invention, but not being part of the invention, are dibasic calcium phosphate dihydrate, polysaccharides, primarily microcrystalline cellulose, lactose, sugars, sorbitol, sucrose, inorganic salts, primarily calcium salts and the like and mixtures thereof. The filler of the present invention is mannitol, silicon dioxide or a mixture thereof, preferably is a mixture thereof.

Examples of binder, as described for understanding the invention, but not being part of the invention, are cellulose or cellulose derivatives, povidone, starch, sucrose, polyethylene glycol, or mixtures thereof. The binder is dissolved in a solvent, for instance, water, an alcohol, or mixtures thereof. In the present invention, the binder solution is hypromellose dissolved in water.

Examples of lubricants, as described for understanding the invention, but not being part of the invention, are sodium stearyl fumarate, calcium stearate talc, stearic acid and mixtures thereof. The lubricant of the present invention is magnesium stearate.

In order to obtain a targetted product profile, quality attributes have been defined and discussed, the manufacturing process has been selected based on these aspects and a control strategy has been defined.

Beginning from the well-known prior art documents and based on experimental studies, multiple formulations were designed to improve dissolution profile and granule characteristics of pharmaceutical compositions comprising ticagrelor.

In an embodiment, not being part of the invention, formulation design

| **Ingredient** | **Formulation** I **Quantity w/w%** |
|---|---|
| Ticagrelor | 25-35 |
| Sorbitol | 35-45 |
| Dibasic calcium phosphate dihydrate | 15-25 |
| Hydroxypropyl cellulose | 1-4 |
| Sodium starch glycolate | 1-2 |
| Magnesium stearate | 1-4 |
| Deionized water | q.s. |
| Coating agent | 1-4 |
| **Film coated tablet** | **100.0** |

Manufacturing method:
1. Ticagrelor, sorbitol, dibasic calcium phosphate dihydrate, hydroxypropyl cellulose and sodium starch glycolate are stirred in high-shear wet granulator for 4 minutes.
2. Sufficient quantity of deionized water was added on the powder blend in step 1 and granulation process is performed with a 10 minute period stirring.
3. Granule obtained in step 2 is dried at a specified temperature.
4. Dried granule is milled and lubricated with magnesium stearate for 3 minutes.
5. Final blend in step 4 is subjected to tablet compression process.
6. Tablets obtained in step 5 were coated with a proper film coating agent.

The formulation design and manufacturing method were very similar to that of innovator drug product. However, the problems observed in ticagrelor drug substance like flowability was poor and stickiness were laso observed in powder blend.

In addition, disintegration analysis was performed on the tablets obtained as per USP Monograph <701>. The result is about 9 to 12 minutes. This value is very high for imediate release solid dosage forms. Moreover, disintegration time result is also the indication for dissolution rate. The longer the disintegration time means the longer the drug release from the formulation. In addition, it is known from the prior arts that in vitro studies have lineer correlation with in vivo drug release.

In order to overcome disintegration problem and obtain a solid pharmaceutical composition with a good chemical ve physical powder blend characteristics, another embodiment was designed.

In an another embodiment, not being part of the invention, use of mannitol was prefered instead of sorbitol. In addition, microcrystalline cellulose was replaced by dibasic calcium phosphate dihydrate not to have stickiness and flowability problem.

| **Ingredient** | **Formulation II Quantity w/w%** |
|---|---|
| Ticagrelor | 25-35 |
| Mannitol | 35-45 |
| Microcrystalline cellulose | 15-25 |
| Hydroxypropyl cellulose | 1-5 |
| Sodium starch glycolate | 1-5 |
| Magnesium stearate | 1-2 |
| Deionized water | q.s. |
| Coating agent | 1-3 |
| **Film coated tablet** | **100.0** |

Manufacturing method:
1. Ticagrelor, mannitol, microcrystalline cellulose, hydroxypropyl cellulose and sodium starch glycolate are stirred in high-shear wet granulator for 5 minutes.
2. Sufficient quantity of deionized water was added on the powder blend in step 1 and granulation process is performed with a 10 minute period stirring.
3. Granule obtained in step 2 is dried at a specified temperature.
4. Dried granule is milled and lubricated with magnesium stearate for 4 minutes.
5. Final blend in step 4 is subjected to tablet compression process.
6. Tablets obtained in step 5 were coated with a proper film coating agent.

It is observed that the flowability problem was overcomed. However, stickiness problem was still ongoing.

Obtained tablets were analyzed for disintegration time, the result is about 1 minute which is good for immediate release solid dosage forms. Thus, in vitro dissolution profile analysis was performed on the tablets tablets.

Dissolution conditions are performed in the following conditions:
- Dissolution medium: Water + 0.2% Polysorbate 80
- Volume of dissolution medium: 900 ml
- Apparatus: Paddle
- Temperature: 37±0.5°C
- Time: 75 minutes
- Rotation speed: 50 rpm

**Table V: Results of dissolution analysis**

| **Time, min.** | **Dissolution Results of Formulation II, %** | **Dissolution Results of Innovator Drug Product, %** |
|---|---|---|
| 10 | 75 | 63 |
| 15 | 85 | 76 |
| 20 | 90 | 82 |
| 30 | 95 | 87 |
| 45 | 98 | 90 |
| 60 | 99 | 91 |
| 75 | 99 | 92 |

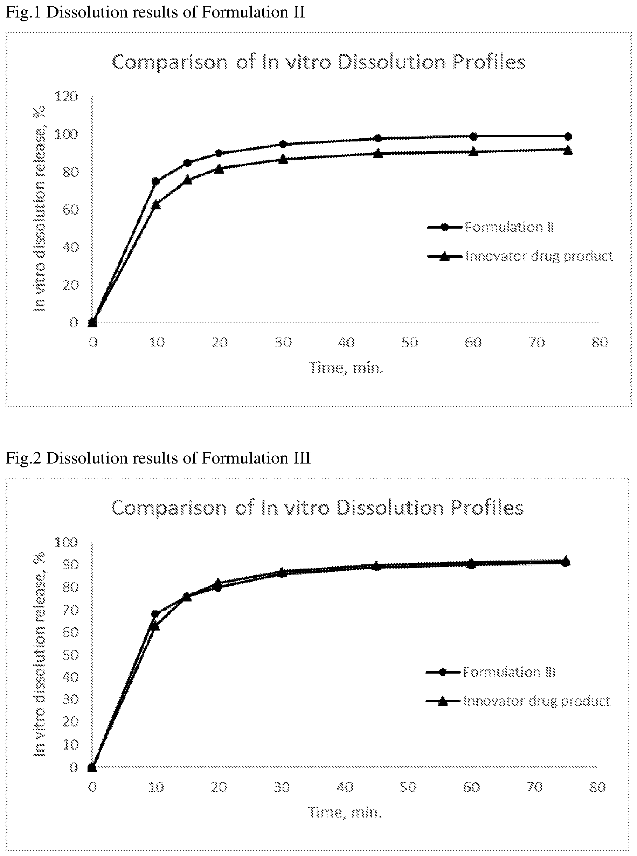

Dissolution results of Formulation II were higher than the dissolution release profile of innovator drug product.(Fig.1)

Comparison of dissolution profiles is shown in the graph. It was observed that the dissolution profile of formulation II presents higher dissolution profile pattern than innovator drug product. Dissoluton study is the major indicator of bioavailability study. The more similar dissolution profile to innovator drug product means closer to achieve pharmaceutical formulation wtih proper in vivo drug release profile.

In another embodiment designated as Formulation III, according to the invention, was designed to lower the dissolution rate which ticagrelor drug substance release under specified conditions. In addition, modifications on existed critical excipients were planned to be made to overcome stickiness problem.

It is known form the prior art, the amount of magnesium stearate has direct impact on dissolution rate. The solvent penetration weight decreased as the percentage of MgSt concentration increased. As a nonsoluble lubricant, MgSt does not allow water to flow through the waxy covering of the powders.

One of critical excipients in the present invention is microcrystalline cellulose for use both in extragranular and intragranular phases taht plays a critical role in compactibility and dissolution rates.

Use of more than one glidants can greatly increase hydrophobicity (the resistance of a particle to absorb water), which lowers the solvent penetration rate (speed of absorption). When a tablet does not allow solvents to penetrate, it will have a slow dissolution, or dissolving rate. Based on this information, an extra diluent silicon dioxide was considered to be used in the next embodiment.

In order to modify the previous formulation (Formulation II), changes made are listed below:
- Increase in Microcrystalline cellulose content
   ∘ Total amount used in the formulation will be increased to get functional effect of not only filler, diluent as well to overcome stickiness problem.
   ∘The amount of Microcrystalline cellulose in the intragranular phase was kept constant. Added amount which makes an increase in Microcrystalline cellulose was located in extragranular phase. It was planned to define the quantity of Microcrystalline cellulose in extragranular phase during manufacturing process in the presence of other excipients.
- Addition of silicon dioxide as diluent
   o Silicon dioxide was planned to be used in the formulation to get functional effect of diluent to overcome stickiness problem. As it is also known from the prior art, silicon dioxide is widely used in pharmaceuticals. Its small particle size and large specific surface area give it desirable flow characteristics that are exploited to improve the flow properties of dry powders in a number of processes such as tableting.

The amounts of critical excipients was considered to be adjusted during manufacturing process.

| **Ingredient** | **Formulation III Quantity w/w%** |
|---|---|
| Ticagrelor | 20-30 |
| Mannitol | 30-40 |
| Microcrystalline cellulose | 30-40 |
| Hydroxypropyl cellulose | 0.1-5 |
| Sodium starch glycolate | 0.1-5 |
| Silicon dioxide | 0.1-2 |
| Magnesium stearate | 0.1-2 |
| Deionized water | q.s. |
| Coating agent | 1-5 |
| **Film coated tablet** | **100.0** |

Manufacturing method:
1. Ticagrelor, mannitol, specified amount of microcrystalline cellulose, specified amount of hydroxypropyl cellulose and sodium starch glycolate are stirred in high-shear wet granulator for 5 minutes.
2. Granulation solution was prepared by dissolving specified amount of hydroxypropyl cellulose in deionized water.
3. Sufficient quantity of granulation solution was added on the powder blend in step 1 and granulation process was performed with a 10 minute period stirring.
4. Granule obtained in step 3 is dried at a specified temperature.
5. Dried granule was sifted and remaining amounts of microcrystalline cellulose and hydroxypropyl cellulose and also silicon dioxide were added and stirred.
6. Obtained granule in step 5 was lubricated with magnesium stearate for 4 minutes.
7. Final blend in step 6 is subjected to tablet compression process.
8. Tablets obtained in step 7 were coated with a proper film coating agent.

Flow properties were good, compression was performed succesfully. Disintegration time analysis and in vitro dissolution study was performed.

Disintegration time was found about 2 minutes.

| **Time, min.** | **Dissolution Results of Formulation III, %** | **Dissolution Results of Innovator Drug Product, %** |
|---|---|---|
| 10 | 68 | 63 |
| 15 | 76 | 76 |
| 20 | 80 | 82 |
| 30 | 86 | 87 |
| 45 | 89 | 90 |
| 60 | 90 | 91 |
| 75 | 91 | 92 |

As it is observed from the Fig.2 , Formulation III presents more similar in vitro dissolution profile with respect to innovator drug product with adjustment of the amounts of critical excipients used in the formulation.

The limiting factor for the appearance of the drug in the blood could be the pure permeability through the intestinal membrane the release from the drug dosage form, or the solubility of the active ingredient. In case of solubility-limited product, the absorption could be governed by physico-chemical characteristics of the API (such as solubility, dissolution rate, particle size, crystal shape, polymorphism) and not by its permeability or formulation.

Based on this information, obtaining proper in vitro dissolution results will lead the formulation to achieve proper in vivo profile.

During wet granulation manufacturing, the amount ratio of Microcrystalline cellulose used in extragranular phase is between 20-30% w/w by total weight of the composition illustrated as Formulation III.

It was surprisingly found that the use of microcrystalline cellulose together with silicon dioxide in the extragranular phase and in a specified range of amount ratio directly affects the in vitro dissolution profile of solid dosage form comprising ticagrelor as active ingredient.

The ratio of microcrystalline cellulose and silicon dioxide in Formulation III is between 35:1 to 45:1.

While the invention has been described with respect to the above specific embodiments, it should be recognized that various modifications and changes may be made to the invention by those skilled in the art which also fall within the scope of the invention as defined by the appended claims.

### Brief description of the drawings:

Fig.1 Dissolution results of Formulation II
Fig.2 Dissolution results of Formulation III

## Claims

1. A pharmaceutical composition comprising ticagrelor and pharmaceutically acceptable excipients manufactured by using wet granulation method wherein;
the composition comprises
- Microcrystalline cellulose used in extragranular phase is between 20-30%w/w by total weight of the formulation,
- Silicon dioxide,
wherein the quantitative composition in w/w % is as stated below:
| | |
|---|---|
| Ticagrelor | 20 - 30 |
| Mannitol | 30 - 40 |
| Microcrystalline cellulose | 30 - 40 |
| Hydroxypropyl cellulose | 0.1 - 5 |
| Sodium starch glycolate | 0.1 - 5 |
| Silicon dioxide | 0.1 - 2 |
| Magnesium stearate | 0.1 - 2 |
| Deionised water | Quantity sufficient |
| Coating agent | 1 - 5 |
wherein together in the extragranular phase in weight ratio of Microcrystalline cellulose : Silicon dioxide is between 35:1 to 45:1.

2. A pharmaceutical composition according to claim 1, wherein the composition is in the form of solid dosage form.

3. A pharmaceutical composition according to any one of the preceding claims, comprises crystalline ticagrelor in the form of polymorph II.

4. A pharmaceutical composition according to claim 3; comprises crystalline ticagrelor having at least X-ray powder diffraction peaks 5.5°, 13.5°, 18.3°, 22.7° and 24.3° (±0.1°2θ).

5. A wet granulation process for preparing a pharmaceutical composition comprises crystalline Ticagrelor as polymorph II, wherein the process comprises the following steps:
a. Ticagrelor, mannitol, specified amount of microcrystalline cellulose, specified amount of hydroxypropyl cellulose and sodium starch glycolate are stirred in high-shear wet granulator for 5 minutes,
b. Granulation solution was prepared by dissolving specified amount of hydroxypropyl cellulose in deionized water,
c. Sufficient quantity of granulation solution was added on the powder blend in step a and granulation process was performed with a 10 minute period stirring,
d. Granule obtained in step c is dried at a specified temperature,
e. Dried granule was sifted and remaining amounts of microcrystalline cellulose and hydroxypropyl cellulose and also silicon dioxide were added and stirred,
f. Obtained granule in step e was lubricated with magnesium stearate for 4 minutes,
g. Final blend in step f is subjected to tablet compression process,
h. Tablets obtained in step g were coated with a proper film coating agent.

## Patentansprüche

1. Pharmazeutische Zusammensetzung, die Ticagrelor und pharmazeutisch akzeptable Hilfsstoffe umfasst, hergestellt unter Verwendung des Nassgranulationsverfahrens, wobei die Zusammensetzung Folgendes umfasst:
- Mikrokristalline Cellulose, die in der extragranularen Phase zwischen 20-30% w/w bezogen auf das Gesamtgewicht der Formulierung liegt,
- Siliciumdioxid,
wobei die quantitative Zusammensetzung in w/w% wie folgt angegeben ist:
Ticagrelor 20 - 30
Mannitol 30 - 40
Mikrokristalline Cellulose 30 - 40
Hydroxypropylcellulose 0,1 - 5
Natriumstärkeglykolat 0,1 - 5
Siliciumdioxid 0,1 - 2
Magnesiumstearat 0,1 - 2
Deionisiertes Wasser Ausreichende Menge
Überzugsmittel 1 - 5
wobei zusammen in der extragranularen Phase das Gewichtsverhältnis von Mikrokristalliner Cellulose:Siliciumdioxid zwischen 35:1 und 45:1 liegt.

2. Pharmazeutische Zusammensetzung nach Anspruch 1, wobei die Zusammensetzung in Form einer festen Darreichungsform vorliegt.

3. Pharmazeutische Zusammensetzung nach einem der vorhergehenden Ansprüche, enthält kristallines Ticagrelor in Form von Polymorph II.

4. Pharmazeutische Zusammensetzung nach Anspruch 3; enthält kristallines Ticagrelor mit mindestens das Pulver-Röntgenbeugungspeaks bei 5,5°, 13,5°, 18,3°, 22,7° und 24,3° (±0,1° 2θ).

5. Ein Nassgranulationsprozess zur Herstellung einer pharmazeutischen Zusammensetzung umfasst kristallines Ticagrelor als Polymorph II, wobei der Prozess die folgenden Schritte umfastt:
a. Ticagrelor, Mannitol, eine festgelegte Menge an mikrokristalliner Cellulose, eine festgelegte Menge an Hydroxypropylcellulose und Natriumstärkeglykolat werden in einem Nassgranulator mit hoher Scherung für 5 Minuten gerührt,
b. Die Granulationslösung wurde durch Auflösen einer festgelegten Menge Hydroxypropylcellulose in deionisiertem Wasser hergestellt,
c. Es wurde eine ausreichende Menge an Granulationslösung auf das Pulvergemisch in Schritt a gegeben, und der Granulationsprozess wurde mit einer 10-minütigen Rührzeit durchgeführt,
d. Das in Schritt c erhaltene Granulat wird bei einer festgelegten Temperatur getrocknet,
e. Das getrocknete Granulat wurde gesiebt, und die verbleibenden Mengen an mikrokristalliner Cellulose, Hydroxypropylcellulose und auch Siliciumdioxid wurden hinzugefügt und gerührt, transalate,
f. Das in Schritt e erhaltenen Granulat wurde 4 Minuten lang mit Magnesiumstearat geschmiert,
g. Die Endmischung in Schritt f wird dem Tablettenpressprozess unterzogen,
h. Die in Schritt g erhaltenen Tabletten wurden mit einem geeigneten Filmüberzugsmittel beschichtet.

## Revendications

1. Une composition pharmaceutique comprenant du ticagrélor et des excipients pharmaceutiquement acceptables, fabriquée à l'aide de la méthode de granulation humide, dans laquelle ;
la composition comprend
- La cellulose microcristalline utilisée dans la phase extragranulaire est comprise entre 20 et 30 % en poids total de la formulation,
- Dioxyde de silicium
où la composition quantitative en % en poids est indiquée ci-dessous :
Ticagrélor 20 - 30
Mannitol 30-40
Cellulose microcristalline 30 - 40
Hydroxypropylcellulose 0,1 - 5
Glycolate d'amidon sodique 0,1 - 5
Dioxyde de silicium 0,1 - 2
Stéarate de magnésium 0,1 - 2
Eau désionisée Quantité suffisante
Agent d'enrobage 1 - 5
où, ensemble dans la phase extragranulaire, le rapport pondéral de cellulose microcristalline : dioxyde de silicium est compris entre 35:1 et 45:1.

2. Composition pharmaceutique selon la revendication 1, dans laquelle la composition se présente sous la forme d'une forme posologique solide.

3. Composition pharmaceutique selon l'une quelconque des revendications précédentes, comprend du ticagrélor cristallin sous la forme du polymorphe II.

4. Composition pharmaceutique selon la revendication 3 ; comprend du ticagrélor cristallin ayant au moins les pics de diffraction des rayons X à 5,5°, 13,5°, 18,3°, 22,7° et 24,3° (±0,1° 2θ).

5. Un procédé de granulation humide pour la préparation d'une composition pharmaceutique comprend du ticagrélor cristallin sous forme de polymorphe II, le procédé comprenant les étapes suivantes :
a. Le ticagrélor, le mannitol, une quantité spécifiée de cellulose microcristalline, une quantité spécifiée de hydroxypropylcellulose et de glycolate d'amidon sodique sont agités dans un granulateur humide à cisaillement élevé pendant 5 minutes,
b. Une solution de granulation a été préparée en dissolvant une quantité spécifiée d'hydroxypropylcellulose dans de l'eau désionisée,
c. Une quantité suffisante de solution de granulation a été ajoutée au mélange de poudre à l'étape a, et le processus de granulation a été réalisé avec un temps d'agitation de 10 minutes,
d. Le granulé obtenu à l'étape c est séché à une température spécifiée,
e. Le granulé séché a été tamisé et les quantités restantes de cellulose microcristalline, d'hydroxypropylcellulose et de dioxyde de silicium ont été ajoutées et agitées,
f. Le granulé obtenu à l'étape e a été lubrifié avec du stéarate de magnésium pendant 4 minutes,
g. Le mélange final à l'étape f est soumis à un processus de compression de comprimés,
h. Les comprimés obtenus à l'étape g ont été enrobés d'un agent d'enrobage approprié.
